# EUROPEAN PATENT SPECIFICATION

(11) **EP 2 186 058 B1**
(45) Date of publication and mention of the grant of the patent: **26.04.2017**
(21) Application number: 08789483.8
(22) Date of filing: 29.07.2008
(51) Int. Cl.: G06T 7/00

(54) **ANATOMICALLY CONSTRAINED IMAGE REGISTRATION**
ANATOMISCH EINGESCHRÄNKTE BILDERFASSUNG
ALIGNEMENT D'IMAGES AVEC CONTRAINTES ANATOMIQUES

(30) Priority: 03.08.2007 EP 07113764
(43) Date of publication of application: 19.05.2010
(73) Proprietor: Koninklijke Philips N.V., 5656 AE Eindhoven (NL); Philips Intellectual Property & Standards GmbH, 20099 Hamburg (DE)
(72) Inventor: PEKAR, Vladimir, NL-5656 AE Eindhoven (NL); BYSTROV, Daniel, NL-5656 AE Eindhoven (NL)
(74) Representative: van Velzen, Maaike Mathilde
(86) International application number: PCT/IB2008/053042
(87) International publication number: WO 2009/019630

(56) References cited:
- WO-A-2005/059831
- WO-A-2006/134565
- US-A1- 2007 014 457
- KAUS ET AL: "Assessment of a Model-Based Deformable Image Registration Approach for Radiation Therapy Planning" INTERNATIONAL JOURNAL OF RADIATION: ONCOLOGY BIOLOGY PHYSICS, PERGAMON PRESS, US, vol. 68, no. 2, 9 May 2007 (2007-05-09), pages 572-580, XP022069508 ISSN: 0360-3016
- ALEJANDRO F FRANGI ET AL: "Automatic Construction of Multiple-Object Three-Dimensional Statistical Shape Models: Application to Cardiac Modeling" IEEE TRANSACTIONS ON MEDICAL IMAGING, IEEE SERVICE CENTER, PISCATAWAY, NJ, US, vol. 21, no. 9, 1 September 2002 (2002-09-01), XP011076353 ISSN: 0278-0062
- COROUGE I ET AL: "Use of a probabilistic shape model for non-linear registration of 3d scattered data" PROCEEDINGS 2001 INTERNATIONAL CONFERENCE ON IMAGE PROCESSING. ICIP 2001. THESSALONIKI, GREECE, OCT. 7 - 10, 2001; [INTERNATIONAL CONFERENCE ON IMAGE PROCESSING], NEW YORK, NY : IEEE, US, vol. 1, 7 October 2001 (2001-10-07), pages 149-152, XP010564818 ISBN: 978-0-7803-6725-8
- JINAH KIM ET AL: "2D/3D non-rigid registration of volumetric CT lung data using thin-plate spline" ENTERPRISE NETWORKING AND COMPUTING IN HEALTHCARE INDUSTRY, 2005. HEAL THCOM 2005. PROCEEDINGS OF 7TH INTERNATIONAL WORKSHOP ON BUSAN, SOUTH KOREA 23-25 JUNE 2005, PISCATAWAY, NJ, USA,IEEE, US, 23 June 2005 (2005-06-23), pages 197-201, XP010830160 ISBN: 978-0-7803-8940-3
- WEINING YUE ET AL: "Locating Large-Scale Craniofacial Feature Points on X-ray Images for Automated Cephalometric Analysis" IMAGE PROCESSING, 2005. ICIP 2005. IEEE INTERNATIONAL CONFERENCE ON GENOVA, ITALY 11-14 SEPT. 2005, PISCATAWAY, NJ, USA,IEEE, vol. 2, 11 September 2005 (2005-09-11), pages 1246-1249, XP010851285 ISBN: 978-0-7803-9134-5
- LORENZ C ET AL: "Generation of Point-Based 3D Statistical Shape Models for Anatomical Objects" COMPUTER VISION AND IMAGE UNDERSTANDING, ACADEMIC PRESS, US, vol. 77, no. 2, 1 February 2000 (2000-02-01), pages 175-191, XP004439301 ISSN: 1077-3142 cited in the application
- BOOKSTEIN F L: "PRINCIPAL WARPS: THIN-PLATE SPLINES AND THE DECOMPOSITION OF DEFORMATIONS" IEEE TRANSACTIONS ON PATTERN ANALYSIS AND MACHINE INTELLIGENCE, IEEE SERVICE CENTER, LOS ALAMITOS, CA, US, vol. 11, no. 6, 1 June 1989 (1989-06-01), pages 567-585, XP000034113 ISSN: 0162-8828 cited in the application

## Description

### FIELD OF THE INVENTION

The invention relates to the field of image registration and more specifically to registration of atlas images with clinical images.

### BACKGROUND OF THE INVENTION

Deformable image registration is the key technology for many clinical applications, for example, when changes in patient's anatomy need to be monitored over time or when comparison of patient's anatomy with an anatomical atlas is required. All currently known deformable registration methods have certain limitations. An overview of image registration methods is given in the article by J. B. Antoine Metz and Max A. Viergever entitled "A survey of medical image registration" in Medical Image Analysis, Vol. 2 (1998) pages 1-37. In particular, standard landmark-based registration techniques require explicit one-to-one landmark correspondences in the registered images. Manual setting of landmarks is a difficult and tedious process, especially in three-dimensional (3-D) data. On the other hand, automated landmark setting methods are mostly application-specific and their design usually requires a considerable effort. Alternatively, intensity-based deformable registration methods require strong assumptions about intensity invariance in the images being registered. These assumptions are often violated.

KAUS ET AL "Assessment of a Model-Based Deformable Image Registration Approach for Radiation Therapy Planning" INTERNATIONAL JOURNAL OF RADIATION: ONCOLOGY BIOLOGY PHYSICS, PERGAMON PRESS, US, VOL. 68 NO. 2, 9 May 2007 (2007-05-09), page 572-580, discloses a surface-model-based deformable image registration system that enables quantitative description of geometrical change in multimodal images with high computational efficiency. Based on the deformation of organ surfaces, a volumetric deformation field is derived using different volumetric elasticity models as alternatives to finite-element modeling.

COROUGE I ET AL: "Use of a probabilistic shape model for non-linear registration of 3d scattered data" PROCEEDINGS 2001 INTERNATIONAL CONFERENCE ON IMAGE PROCESSING. ICIP 2001. THESSALONIKI, GREECE, OCT. 7-10, 200; vol. 1, 7 October 2001 (2001-10-07), pages 149-152 discloses a local and non-linear interindividual fusion scheme of anatomical and functional data, based both on a satistical modeling of anatomical structures, the cortical sulci, and on the use of the thin-plate spline method.

ALEJANDRO F FRANGI ET AL:"Automatic construction of Multiple-Object Three-Dimensional Statistical Shape Models: Application to Cardiac Modeling", IEEE TRANSACTIONS ON MEDICAL IMAGING, IEEE SERVICE CENTER, PISCATAWAY, NJ, US, vol. 21, no.9, 1 September 2002 discloses a method for the generation of landmarks for three-dimensional (3-D) shapes and the construction of the corresponding 3-D statistical shape models. Automatic landmarking of a set of manual segmentations from a class of shapes is achieved by 1) construction of an atlas of the class, 2) automatic extraction of the landmarks from the atlas, and 3) subsequent propagation of these landmarks to each example shape via a volumetric nonrigid registration technique using multiresolution B-spline deformations.

WO 2005/059831 A discloses a non-rigid image registration by an iterative refinement process based on a combination of landmarks with similarity values.

### SUMMARY OF THE INVENTION

It would be advantageous to have a system capable of registering an objective image with an atlas image, which does not require explicit landmark setting.

To better address this issue, in an aspect of the invention, a system for registering an atlas image from an atlas of multidimensional images with an objective image is provided, the atlas image having been computed based on a plurality of training images, and the system comprising:
- a generation unit for generating a candidate transformation based on a candidate transformation field for transforming a first region of the atlas image, wherein the first region comprises a plurality of landmarks, each training image from the plurality of training images comprises the plurality of landmarks, and wherein the candidate transformation field is defined by elementary transformation fields each being derived from displacements of i) the plurality of landmarks in a respective one of the plurality of training images, relative to ii) landmark position in the atlas image;
- a transformation unit for transforming the first region of the atlas image using the candidate transformation;
- a similarity unit for computing a measure of similarity of the transformed first region of the atlas image and a corresponding first region of the objective image;
- an evaluation unit for evaluating the candidate transformation using a criterion based on the computed measure of similarity and determining an optimal transformation based on the evaluation of the candidate transformation;
- an extension unit for extending the optimal transformation of the first region of the atlas image to a second region of the atlas image, wherein the second region comprises the first region, thereby creating a registration transformation; and
- a registration unit for transforming the second region using the registration transformation, thereby registering the atlas image with the objective image,
wherein the candidate transformation is a linear combination of the elementary transformation fields, and
wherein the each elementary transformation field is computed based on the principal component analysis of said displacements of the plurality of landmarks.

The first region is typically defined in such a way that it comprises atlas image features which are relatively easy to match with the corresponding multidimensional image features. When the measure of similarity of the transformed first region of the atlas image to a corresponding first region of the objective image satisfies the evaluation criterion, e.g., when this measure of similarity is substantially a maximum of a similarity function for computing the similarity measure, the candidate transformation becomes the optimal transformation. The optimal transformation of the first region may now be extended to a second region, e.g., to the atlas image area or volume. This extension of the optimal transformation, i.e., the registration transformation, is used by the registration unit to register the atlas image with the objective image. Advantageously, the system does not require setting landmark positions in the objective image. A further advantage of the system is that since the similarity measure is computed locally, i.e., in the first region of the atlas image, the registration is fast and thus attractive for clinical use.

In an embodiment of the system, the first region comprises a plurality of landmarks. The landmarks are atlas image features which are easy to identify in the objective image.

In an embodiment of the system, the atlas image is computed based on a plurality of training images, each training image from the plurality of training images comprising the plurality of landmarks. For example, the atlas image may be computed by determining atlas landmark positions in the atlas image based on landmark positions in the plurality of training images; registering the plurality of training images with landmark positions in the atlas image; and computing the mean of the registered plurality of training images, thereby computing the atlas image. Advantageously, such an atlas image is typically sharp around the landmarks, thus having well defined atlas image features, while it may be fuzzy away from the landmarks. The first region of the atlas image may be a union of small sharp landmark neighborhoods, each neighborhood comprising a feature from the plurality of features.

In an embodiment of the system, the candidate transformation of the atlas image is generated based on a candidate transformation field defined by elementary transformation fields, wherein each elementary transformation field is derived from displacements of landmarks comprised in a training image from the plurality of training images relative to the atlas landmark positions. Such elementary transformation fields capture typical variations of feature positions.

In an embodiment of the system, each elementary transformation field is based on the principal component analysis of the displacements of landmarks comprised in the training image from the plurality of training images relative to the atlas image. Using elementary transformations based on the principal analysis allows capturing characteristic modes of displacements.

In an embodiment of the system, the system further comprises an atlas unit for constructing the atlas of multidimensional images. The atlas may comprise the atlas image data and the elementary transformation fields. This allows a user to build a customized system to meet the user requirements.

In a further aspect of the invention, the system according to the invention is comprised in an image acquisition apparatus.

In a further aspect of the invention, the system according to the invention is comprised in a workstation.

In a further aspect of the invention, a method of registering an atlas image from an atlas of multidimensional images with an objective image is provided, the atlas image having been computed based on a plurality of training images, and the method comprising:
- a generation step for generating a candidate transformation based on a candidate transformation field for transforming a first region of the atlas image, wherein the first region comprises a plurality of landmarks, each training image from the plurality of training images comprises the plurality of landmarks, and wherein the candidate transformation field is defined by elementary transformation fields each being derived from displacements of i) the plurality of landmarks in a respective one of the plurality of training images, relative to ii) the plurality of landmarks in the atlas image;
- a transformation step for transforming the first region of the atlas image using the candidate transformation;
- a similarity step for computing a measure of similarity of the transformed first region of the atlas image and a corresponding first region of the objective image;
- an evaluation step for evaluating the candidate transformation using a criterion based on the computed measure of similarity and determining an optimal transformation based on the evaluation of the candidate transformation;
- an extension step for extending the optimal transformation of the first region of the atlas image to a second region of the atlas image, wherein the second region comprises the first region, thereby creating a registration transformation; and
- a registration step for transforming the second region using the registration transformation, thereby registering the atlas image with the objective image,
wherein the candidate transformation is a linear combination of the elementary transformation fields, and
wherein the each elementary transformation field is computed based on the principal component analysis of said displacements of the plurality of landmarks.

In a further aspect of the invention, a computer program product to be loaded by a computer arrangement is provided, the computer program product comprising instructions for registering an atlas image from an atlas of multidimensional images with an objective image, the atlas image having been computed based on a plurality of training images, the computer arrangement comprising a processing unit and a memory, the computer program product, after being loaded, providing said processing unit with the capability to carry out the tasks of:
- generating a candidate transformation based on a candidate transformation field for transforming a first region of the atlas image, wherein the first region comprises a plurality of landmarks, each training image from the plurality of training images comprises the plurality of landmarks, and wherein the candidate transformation field is defined by elementary transformation fields each being derived from displacements of i) the plurality of landmarks in a respective one of the plurality of training images, relative to ii) the plurality of landmarks in the atlas image;
- transforming the first region of the atlas image using the candidate transformation;
- computing a measure of similarity of the transformed first region of the atlas image and a corresponding first region of the objective image;
- evaluating the candidate transformation using a criterion based on the computed measure of similarity and determining an optimal transformation based on the evaluation of the candidate transformation;
- extending the optimal transformation of the first region of the atlas image to a second region of the atlas image, wherein the second region comprises the first region, thereby creating a registration transformation; and
- transforming the second region using the registration transformation, thereby registering the atlas image with the objective image,
wherein the candidate transformation is a linear combination of the elementary transformation fields, and
wherein the each elementary transformation field is computed based on the principal component analysis of said displacements of the plurality of landmarks.

It will be appreciated by those skilled in the art that two or more of the above-mentioned embodiments, implementations, and/or aspects of the invention may be combined in any way deemed useful.

Modifications and variations of the image acquisition apparatus, of the workstation, of the method, and/or of the computer program product, which correspond to the described modifications and variations of the system, can be carried out by a skilled person on the basis of the present description.

The skilled person will appreciate that the method may be applied to multidimensional image data, e.g., to 2-dimensional, 3-dimensional, or 4-dimensional images, acquired by various acquisition modalities such as, but not limited to, standard X-ray Imaging, Computed Tomography (CT), Magnetic Resonance Imaging (MRI), Ultrasound (US), Positron Emission Tomography (PET), Single Photon Emission Computed Tomography (SPECT), and Nuclear Medicine (NM).

### BRIEF DESCRIPTION OF THE DRAWINGS

These and other aspects of the invention will become apparent from and will be elucidated with respect to the implementations and embodiments described hereinafter and with reference to the accompanying drawings, wherein:
Fig. 1 schematically shows a block diagram of an exemplary embodiment of the system;
Fig. 2 shows exemplary landmark positions in an exemplary atlas image and in three exemplary objective images;
Fig. 3 shows a flowchart of an exemplary implementation of the method;
Fig. 4 schematically shows an exemplary embodiment of the image acquisition apparatus; and
Fig. 5 schematically shows an exemplary embodiment of the workstation.
Identical reference numerals are used to denote similar parts throughout the Figures.

### DETAILED DESCRIPTION OF EMBODIMENTS

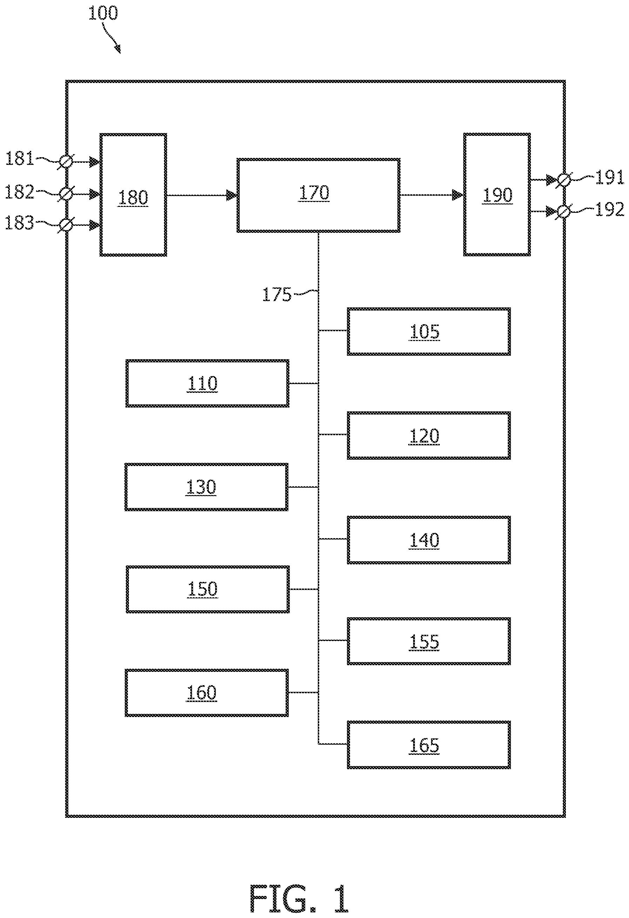

Fig. 1 schematically shows a block diagram of an exemplary embodiment of the system 100 for registering an atlas image from an atlas of multidimensional images with an objective image, the system comprising:
- a generation unit 105 for generating a candidate transformation for transforming a first region of the atlas image;
- a transformation unit 110 for transforming the first region of the atlas image using the candidate transformation;
- a similarity unit 120 for computing a measure of similarity of the transformed first region of the atlas image and a corresponding first region of the objective image;
- an evaluation unit 130 for evaluating the candidate transformation using a criterion based on the computed measure of similarity and determining an optimal transformation based on the evaluation of the candidate transformation;
- an extension unit 140 for extending the optimal transformation of the first region of the atlas image to a second region of the atlas image, wherein the second region comprises the first region, thereby creating a registration transformation; and
- a registration unit 150 for transforming the second region using the registration transformation, thereby registering the atlas image with the objective image.

The exemplary embodiment of the system 100 further comprises the following units:
- an atlas unit 155 for constructing the atlas of multidimensional images
- a control unit 160 for controlling the workflow in the system 100
- a user interface 165 for communicating with a user of the system 100; and
- a memory unit 170 for storing data.

In an embodiment of the system 100, there are three input connectors 181, 182 and 183 for the incoming data. The first input connector 181 is arranged to receive data coming in from a data storage means such as, but not limited to, a hard disk, a magnetic tape, a flash memory, or an optical disk. The second input connector 182 is arranged to receive data coming in from a user input device such as, but not limited to, a mouse or a touch screen. The third input connector 183 is arranged to receive data coming in from a user input device such as a keyboard. The input connectors 181, 182 and 183 are connected to an input control unit 180.

In an embodiment of the system 100, there are two output connectors 191 and 192 for the outgoing data. The first output connector 191 is arranged to output the data to a data storage means such as a hard disk, a magnetic tape, a flash memory, or an optical disk. The second output connector 192 is arranged to output the data to a display device. The output connectors 191 and 192 receive the respective data via an output control unit 190.

The skilled person will understand that there are many ways to connect input devices to the input connectors 181, 182 and 183 and the output devices to the output connectors 191 and 192 of the system 100. These ways comprise, but are not limited to, a wired and a wireless connection, a digital network such as, but not limited to, a Local Area Network (LAN) and a Wide Area Network (WAN), the Internet, a digital telephone network, and an analog telephone network.

In an embodiment of the system 100, the system 100 comprises a memory unit 170. The system 100 is arranged to receive input data from external devices via any of the input connectors 181, 182, and 183 and to store the received input data in the memory unit 170. Loading the input data into the memory unit 170 allows quick access to relevant data portions by the units of the system 100. The input data may comprise, for example, the objective image data. The memory unit 170 may be implemented by devices such as, but not limited to, a Random Access Memory (RAM) chip, a Read Only Memory (ROM) chip, and/or a hard disk drive and a hard disk. The memory unit 170 may be further arranged to store the output data. The output data may comprise, for example, the atlas image registered with the objective image. The memory unit 170 may be also arranged to receive data from and deliver data to the units of the system 100 comprising the generation unit 105, the transformation unit 110, the similarity unit 120, the evaluation unit 130, the extension unit 140, the registration unit 150, the control unit 160, and the user interface 165, via a memory bus 175. The memory unit 170 is further arranged to make the output data available to external devices via any of the output connectors 191 and 192. Storing data from the units of the system 100 in the memory unit 170 may advantageously improve performance of the units of the system 100 as well as the rate of transfer of the output data from the units of the system 100 to external devices.

Alternatively, the system 100 may comprise no memory unit 170 and no memory bus 175. The input data used by the system 100 may be supplied by at least one external device, such as an external memory or a processor, connected to the units of the system 100. Similarly, the output data produced by the system 100 may be supplied to at least one external device, such as an external memory or a processor, connected to the units of the system 100. The units of the system 100 may be arranged to receive the data from each other via internal connections or via a data bus.

In an embodiment of the system 100, the system 100 comprises a control unit 160 for controlling the workflow in the system 100. The control unit may be arranged to receive control data from and provide control data to the units of the system 100. For example, after generating the candidate transformation, the generation unit 105 may be arranged to pass control data "the candidate transformation is generated" to the control unit 160 and the control unit 160 may be arranged to provide control data "transform the first region" to the transformation unit 110, thereby requesting the transformation unit 110 to transform the first region. Alternatively, a control function may be implemented in another unit of the system 100.

In an embodiment of the system 100, the system 100 comprises a user interface 165 for communicating with the user of the system 100. The user interface 165 may be arranged to obtain a user input, e.g., a request for displaying a view computed from the registered atlas image. The skilled person will understand that more functions may be advantageously implemented in the user interface 165 of the system 100.

In an embodiment of the system 100, the atlas image comprises a plurality of landmarks. The atlas image may be manually or automatically annotated by setting landmarks in structures that are present in the atlas image. For example, prominent structures, such as intervertebral disks, may be marked in an atlas image describing the spine. Examples of landmarks in atlas images describing a heart include, but are not limited to, segments and branching points of main cardiac arteries and heart apex. The first region of the atlas image is often determined by the distribution of landmarks. For example, the first region may be a union of neighborhoods, e.g., spherical neighborhoods, of landmarks.

In an embodiment of the system 100, the atlas image is constructed based on a plurality of training images. Each training image may be manually or automatically annotated by setting landmarks in structures that are present in the training image. In an embodiment of the system 100, the training images are scaled and aligned using, for example, the Procrustes analysis. Procrustes analysis is described in a book by I. L. Dryden and K. V. Mardia entitled "Statistical Shape Analysis", John Wiley & Sons; Chichester, UK, 1998. The mean position of each landmark is computed based on the positions of the landmark in the plurality of training images. The training images are brought to a common reference based on the mean landmark positions. For example, each training image is transformed using a transformation defined by a field of displacements of landmark positions in the training image relative to the mean landmark positions. The transformation of the training image may be defined using thin-plate interpolation of the field of displacements. Constructing a vector field using the thin-plate-spline interpolation of a sparse vector field is described in an article by F. L. Bookstein entitled "Principal warps: Thin-plate splines and the decomposition of deformations" in IEEE Transactions on Pattern Analysis and Machine Intelligence, Vol. 11, pages 567-585, 1989, hereafter referred to as Ref. 1. The mean of the transformed training images defines the atlas image. Intensity values of the atlas image constructed as described above are guaranteed to well describe image features near the landmarks. In other regions, image features may be fuzzy.

The generating unit 105 is arranged to generate a candidate transformation for transforming a first region of the atlas image. The skilled person will appreciate that various transformations may be employed by the generating unit including rigid, affine, piece-wise affine and elastic transformations.

In an embodiment of the system 100, the candidate transformation of the atlas image is generated based on a candidate transformation field defined by elementary transformation fields, wherein each elementary transformation field is derived from displacements of landmarks comprised in a training image from the plurality of training images relative to landmark positions in the atlas image. The elementary transformation field may be computed using the principal component analysis of the displacements of landmarks comprised in training images from the plurality of training images relative to the atlas landmark positions. Using principal component analysis to describe the variability of landmark positions is described, for example, in the article "Generation of point-based 3D statistical shape models for anatomical objects" by Cristian Lorenz and Nils Krahnstöver, in Computer Vision and Image Understanding 77(2), 2000, pages 175-191.

In an embodiment of the system 100, the candidate transformation is a linear combination of the elementary transformation fields. The transformation unit 110 is arranged for transforming the first region of the atlas image using the candidate transformation. The similarity unit 120 is arranged for computing a measure of similarity of the transformed first region of the atlas image and a corresponding first region of the objective image. The corresponding first region of the objective image is a region of the objective image of which the area or volume is defined by the transformed first region of the atlas image, and of which gray values are defined by the gray values of the objective image. The measure of similarity may be based on template matching or matching voxels of the transformed atlas image and objective image, randomly selected in the first region of the objective image, e.g., around each landmark. Hereafter the term volume refers to a multidimensional volume, e.g., a 2-dimensional (i.e., area), 3-dimensional, or 4-dimensional volume. Similarly, the term voxel refers to a multidimensional voxel, e.g., a 2-dimensional (i.e., pixel), 3-dimensional, or 4-dimensional voxel. The measure of similarity of the transformed first region of the atlas image and the corresponding first region of the objective image depends on the candidate transformation, e.g., on the coefficients of the linear combination of elementary fields. The evaluation unit 130 is arranged for evaluating the candidate transformation using a criterion based on the computed measure of similarity and determining an optimal transformation based on the evaluation of the candidate transformation. The criterion may be that the similarity measure is equal to or greater than a pre-determined, system-determined, or user-determined threshold. Another criterion may be that the similarity measure is a maximum in a parameter space, e.g., in the space of coefficients of the linear combination of the elementary fields.

If the candidate transformation satisfies the criterion, it becomes an optimal transformation and the extension unit 140 is arranged to extend the optimal transformation of the first region of the atlas image to a second region of the atlas image, wherein the second region comprises the first region, thereby creating a registration transformation. The extension may be based on the thin-plate interpolation of the candidate transformation field defined by the linear combination of the elementary transformation fields, as described in Ref. 1. The second region may comprise the whole volume of the atlas image. The registration unit 150 is arranged to transform the second region using the registration transformation, thereby registering the atlas image with the objective image.

If the candidate transformation does not satisfy the criterion, the generation unit 105 is arranged to generate another candidate transformation for evaluation by the evaluation unit 130 based on the measure of similarity computed by the similarity unit 120. The generating unit 105 may receive an input from the evaluation unit for generating the candidate transformation. The skilled person will understand that there are many possible embodiments of the system 100. The generation unit 105, the transformation unit 110, the similarity unit 120, and the evaluation unit 130 may be arranged to implement an algorithm for optimizing the candidate transformation, i.e., for finding the optimal transformation. Such algorithms comprise, but are not limited to, the steepest ascent or descent path algorithm, conjugate gradient algorithm, random search algorithm, and simplex method algorithm.

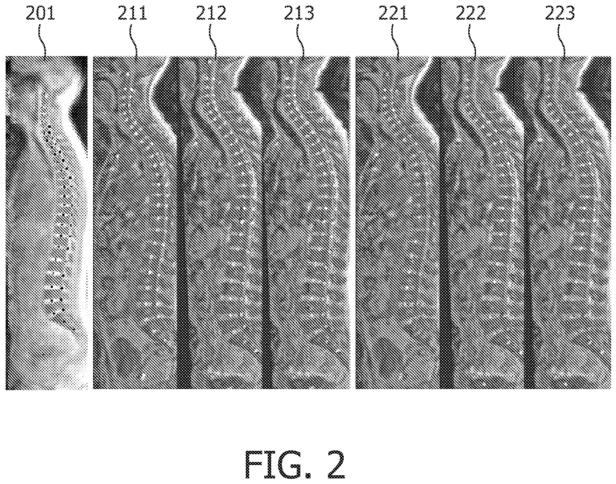

Fig. 2 shows exemplary landmark positions in an exemplary atlas image and in three exemplary objective images of the spine. Image 201 shows the exemplary atlas image of the spine. The black dots indicate landmark positions in the exemplary atlas image. Images 211, 212 and 213 show three objective images of the spine with landmark positions computed by the system 100. The landmark positions are represented by white dots along the spine. Images 221, 222 and 223 show the same three objective images with landmark positions manually determined by a medical expert. The landmark positions are represented by white dots along the spine. A comparison of pairs of images 211 and 221, 212 and 222, and 213 and 223 illustrates that the registration of the exemplary atlas image 201 and the objective images is capable of producing satisfactory results.

In an examplary application of the system 100, the system 100 is used for registering an anatomical atlas with 3-dimensional scout images for automated image acquisition planning.

The skilled person will further understand that the system 100 described in the current document may be a valuable tool for assisting a physician in many aspects of her/his job.

The skilled person will further understand that other embodiments of the system 100 are also possible. It is possible, among other things, to redefine the units of the system and to redistribute their functions. Although the described embodiments apply to medical images, other applications of the system, outside the medical domain, are also possible.

The units of the system 100 may be implemented using a processor. Normally, their functions are performed under the control of a software program product. During execution, the software program product is normally loaded into a memory, like a RAM, and executed from there. The program may be loaded from a background memory, such as a ROM, hard disk, or magnetic and/or optical storage, or may be loaded via a network like the Internet. Optionally, an application-specific integrated circuit may provide the described functionality.

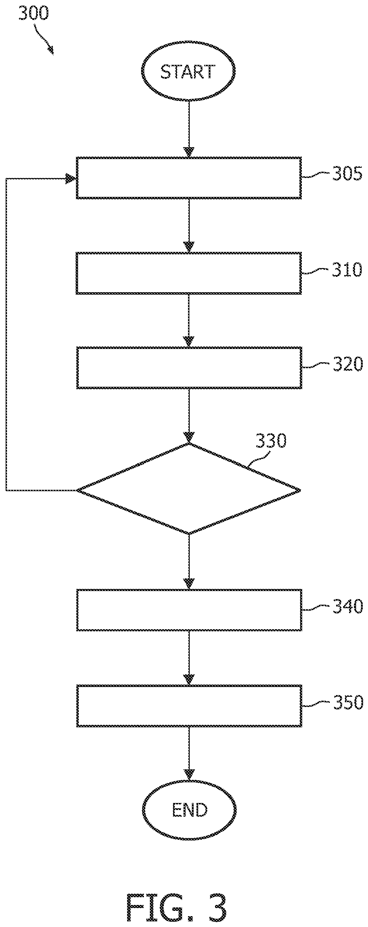

Fig. 3 shows a flowchart of an exemplary implementation of the method 300 of registering an atlas image from an atlas of multidimensional images with an objective image. The method 300 begins with a generation step 305 for generating a candidate transformation for transforming a first region of the atlas image. After the generation step 305, the method 300 continues to a transformation step 310 for transforming the first region of the atlas image using the candidate transformation. After the transformation step 310, the method 300 continues to a similarity step 320 for computing a measure of similarity of the transformed first region of the atlas image and a corresponding first region of the objective image. After the similarity step 320, the method 300 continues to an evaluation step 330 for evaluating the candidate transformation using a criterion based on the computed measure of similarity and determining an optimal transformation based on the evaluation of the candidate transformation. After the evaluation step 330, the method 300 continues to an extension step 340 for extending the optimal transformation of the first region of the atlas image to a second region of the atlas image, wherein the second region comprises the first region, thereby creating a registration transformation, or to the generation step 305, depending on the outcome of the evaluation of the candidate transformation. If the criterion based on the computed measure of similarity is satisfied, the method 300 continues to the generation step 305. Otherwise the method continues to the extension step 340. After the extension step 340, the method continues to a registration step 350 for transforming the second region using the registration transformation, thereby registering the atlas image with the objective image. After the registration step 350 the method terminates.

The skilled person may change the order of some steps or perform some steps concurrently using threading models, multi-processor systems or multiple processes without departing from the concept as intended by the present invention. Optionally, two or more steps of the method 400 of the current invention may be combined into one step. Optionally, a step of the method 400 of the current invention may be split into a plurality of steps.

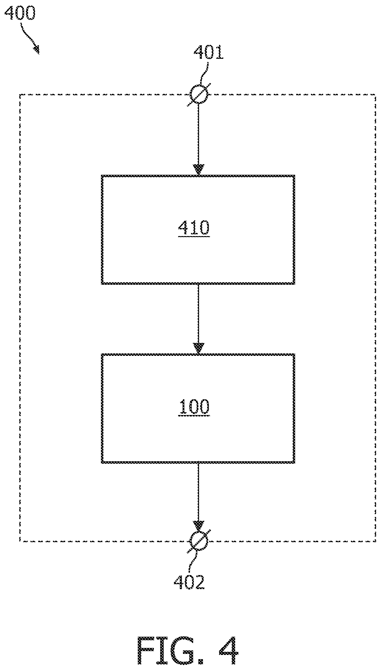

Fig. 4 schematically shows an exemplary embodiment of the image acquisition apparatus 400 employing the system 100, said image acquisition apparatus 400 comprising a CT image acquisition unit 410 connected via an internal connection with the system 100, an input connector 401, and an output connector 402. This arrangement advantageously increases the capabilities of the image acquisition apparatus 400, providing said image acquisition apparatus 400 with advantageous capabilities of the system 100.

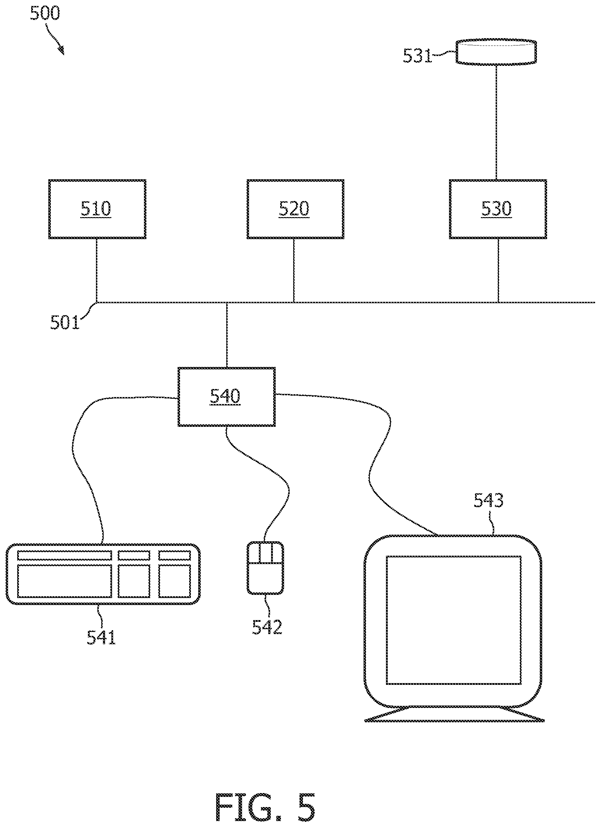

Fig. 5 schematically shows an exemplary embodiment of the workstation 500. The workstation comprises a system bus 501. A processor 510, a memory 520, a disk input/output (I/O) adapter 530, and a user interface (UI) 540 are operatively connected to the system bus 501. A disk storage device 531 is operatively coupled to the disk I/O adapter 530. A keyboard 541, a mouse 542, and a display 543 are operatively coupled to the UI 540. The system 100 of the invention, implemented as a computer program, is stored in the disk storage device 531. The workstation 500 is arranged to load the program and input data into memory 520 and execute the program on the processor 510. The user can input information to the workstation 500 using the keyboard 541 and/or the mouse 542. The workstation is arranged to output information to the display device 543 and/or to the disk 531. The skilled person will understand that there are numerous other embodiments of the workstation 500 known in the art and that the present embodiment serves the purpose of illustrating the invention and must not be interpreted as limiting the invention to this particular embodiment.

It should be noted that the above-mentioned embodiments illustrate rather than limit the invention and that those skilled in the art will be able to design alternative embodiments without departing from the scope of the appended claims. In the claims, any reference signs placed between parentheses shall not be construed as limiting the claim. The word "comprising" does not exclude the presence of elements or steps not listed in a claim or in the description. The word "a" or "an" preceding an element does not exclude the presence of a plurality of such elements. The invention can be implemented by means of hardware comprising several distinct elements and by means of a programmed computer. In the system claims enumerating several units, several of these units can be embodied by one and the same item of hardware or software. The usage of the words first, second and third, etc., does not indicate any ordering. These words are to be interpreted as names.

## Claims

1. A system (100) for registering an atlas image from an atlas of multidimensional images with an objective image, the atlas image having been computed based on a plurality of training images, and the system comprising:
- a generation unit (105) for generating a candidate transformation based on a candidate transformation field for transforming a first region of the atlas image, wherein the first region comprises a plurality of landmarks, each training image from the plurality of training images comprises the plurality of landmarks, and wherein the candidate transformation field is defined by elementary transformation fields each being derived from displacements of i) the plurality of landmarks in a respective one of the plurality of training images, relative to ii) landmark position in the atlas image, wherein the candidate transformation is a linear combination of the elementary transformation fields, and wherein the each elementary transformation field is computed based on the principal component analysis of said displacements of the plurality of landmarks;
- a transformation unit (110) for transforming the first region of the atlas image using the candidate transformation;
- a similarity unit (120) for computing a measure of similarity of the transformed first region of the atlas image and a corresponding first region of the objective image;
- an evaluation unit (130) for evaluating the candidate transformation using a criterion based on the computed measure of similarity and determining an optimal transformation based on the evaluation of the candidate transformation;
- an extension unit (140) for extending the optimal transformation of the first region of the atlas image to a second region of the atlas image, wherein the second region comprises the first region, thereby creating a registration transformation; and
- a registration unit (150) for transforming the second region using the registration transformation, thereby registering the atlas image with the objective image.

2. A system (100) as claimed in claim 1, wherein the computed measure of similarity is a maximum in a parameter space defined by coefficients of the linear combination of the elementary fields.

3. A system (100) as claimed in claim 1, further comprising an atlas unit (155) for constructing the atlas of multidimensional images.

4. An image acquisition apparatus (400) comprising a system (100) as claimed in claim 1.

5. A workstation (500) comprising a system (100) as claimed in claim 1.

6. A method (300) of registering an atlas image from an atlas of multidimensional images with an objective image, the atlas image having been computed based on a plurality of training images, and the method comprising:
- a generation step (305) for generating a candidate transformation based on a candidate transformation field for transforming a first region of the atlas image, wherein the first region comprises a plurality of landmarks, each training image from the plurality of training images comprises the plurality of landmarks, and wherein the candidate transformation field is defined by elementary transformation fields each being derived from displacements of i) the plurality of landmarks in a respective one of the plurality of training images, relative to ii) landmark position in the atlas image, wherein the candidate transformation is a linear combination of the elementary transformation fields, and wherein the each elementary transformation field is computed based on the principal component analysis of said displacements of the plurality of landmarks;
- a transformation step (310) for transforming the first region of the atlas image using the candidate transformation;
- a similarity step (320) for computing a measure of similarity of the transformed first region of the atlas image and a corresponding first region of the objective image;
- an evaluation step (330) for evaluating the candidate transformation using a criterion based on the computed measure of similarity and determining an optimal transformation based on the evaluation of the candidate transformation;
- an extension step (340) for extending the optimal transformation of the first region of the atlas image to a second region of the atlas image, wherein the second region comprises the first region, thereby creating a registration transformation; and
- a registration step (350) for transforming the second region using the registration transformation, thereby registering the atlas image with the objective image.

7. A computer program product to be loaded by a computer arrangement, comprising instructions for registering an atlas image from an atlas of multidimensional images with an objective image, the atlas image having been computed based on a plurality of training images, the computer arrangement comprising a processing unit and a memory, the computer program product, after being loaded, providing said processing unit with the capability to carry out the tasks of:
- generating a candidate transformation based on a candidate transformation field for transforming a first region of the atlas image, wherein the first region comprises a plurality of landmarks, each training image from the plurality of training images comprises the plurality of landmarks, and wherein the candidate transformation field is defined by elementary transformation fields each being derived from displacements of i) the plurality of landmarks in a respective one of the plurality of training images, relative to ii) landmark position in the atlas image, wherein the candidate transformation is a linear combination of the elementary transformation fields, and wherein the each elementary transformation field is computed based on the principal component analysis of said displacements of the plurality of landmarks;
- transforming the first region of the atlas image using the candidate transformation;
- computing a measure of similarity of the transformed first region of the atlas image and a corresponding first region of the objective image;
- evaluating the candidate transformation using a criterion based on the computed measure of similarity and determining an optimal transformation based on the evaluation of the candidate transformation;
- extending the optimal transformation of the first region of the atlas image to a second region of the atlas image, wherein the second region comprises the first region, thereby creating a registration transformation; and
- transforming the second region using the registration transformation, thereby registering the atlas image with the objective image.

## Patentansprüche

1. System (100) zum Registrieren eines Atlasbilds aus einem Atlas multidimensionaler Bilder mit einem gegenständlichen Bild, wobei das Atlasbild basierend auf einer Vielzahl von Trainingsbildern berechnet wurde, und das System Folgendes umfasst:
- eine Erzeugungseinheit (105) zum Erzeugen einer in Frage kommenden Transformation basierend auf einem in Frage kommenden Transformationsfeld zum Transformieren einer ersten Region des Atlasbilds, wobei die erste Region eine Vielzahl von Orientierungspunkten umfasst, jedes Trainingsbild aus der Vielzahl von Trainingsbildern die Vielzahl von Orientierungspunkten umfasst, und wobei das in Frage kommende Transformationsfeld durch elementare Transformationsfelder definiert wird, die jeweils abgeleitet werden von Verlagerungen von i) der Vielzahl von Orientierungspunkten in einem jeweiligen der Vielzahl von Trainingsbildern relativ zu ii) der Orientierungspunktposition in dem Atlasbild, wobei die in Frage kommende Transformation eine lineare Kombination der elementaren Transformationsfelder ist, und wobei jedes elementare Transformationsfeld basierend auf der Hauptkomponentenanalyse der genannten Verlagerungen der Vielzahl von Orientierungspunkten berechnet wird;
- eine Transformationseinheit (110) zum Transformieren der ersten Region des Atlasbilds unter Verwendung der in Frage kommenden Transformation;
- eine Ähnlichkeitseinheit (120) zum Berechnen eines Ähnlichkeitsmaßes der transformierten ersten Region des Atlasbilds und einer entsprechenden ersten Region des gegenständlichen Bilds;
- eine Auswertungseinheit (130) zum Auswerten der in Frage kommenden Transformation unter Verwendung eines auf dem berechneten Ähnlichkeitsmaß basierenden Kriteriums und Ermitteln einer optimalen Transformation basierend auf der Auswertung der in Frage kommenden Transformation;
- eine Erweiterungseinheit (140) zum Erweitern der optimalen Transformation der ersten Region des Atlasbilds auf eine zweite Region des Atlasbilds, wobei die zweite Region die erste Region umfasst, wodurch eine Registrierungstransformation geschaffen wird; und
- eine Registrierungseinheit (150) zum Transformieren der zweiten Region unter Verwendung der Registrierungstransformation, wodurch das Atlasbild mit dem gegenständlichen Bild registriert wird.

2. System (100) nach Anspruch 1, wobei das berechnete Ähnlichkeitsmaß ein Maximum in einem Parameterraum ist, der durch Koeffizienten der linearen Kombination der elementaren Felder definiert wird.

3. System (100) nach Anspruch 1, weiterhin umfassend eine Atlaseinheit (155) zum Konstruieren des Atlas mehrdimensionaler Bilder.

4. Bilderfassungsgerät (400) umfassend ein System (100) nach Anspruch 1.

5. Workstation (500) umfassend ein System (100) nach Anspruch 1.

6. Verfahren (300) zum Registrieren eines Atlasbilds aus einem Atlas multidimensionaler Bilder mit einem gegenständlichen Bild, wobei das Atlasbild basierend auf einer Vielzahl von Trainingsbildern berechnet wurde, und das Verfahren Folgendes umfasst:
- einen Erzeugungsschritt (305) zum Erzeugen einer in Frage kommenden Transformation basierend auf einem in Frage kommenden Transformationsfeld zum Transformieren einer ersten Region des Atlasbilds, wobei die erste Region eine Vielzahl von Orientierungspunkten umfasst, jedes Trainingsbild aus der Vielzahl von Trainingsbildern die Vielzahl von Orientierungspunkten umfasst, und wobei das in Frage kommende Transformationsfeld durch elementare Transformationsfelder definiert wird, die jeweils abgeleitet werden von Verlagerungen von i) der Vielzahl von Orientierungspunkten in einem jeweiligen der Vielzahl von Trainingsbildern relativ zu ii) der Orientierungspunktposition in dem Atlasbild, wobei die in Frage kommende Transformation eine lineare Kombination der elementaren Transformationsfelder ist, und wobei jedes elementare Transformationsfeld basierend auf der Hauptkomponentenanalyse der genannten Verlagerungen der Vielzahl von Orientierungspunkten berechnet wird;
- einen Transformationsschritt (310) zum Transformieren der ersten Region des Atlasbilds unter Verwendung der in Frage kommenden Transformation;
- einen Ähnlichkeitsschritt (320) zum Berechnen eines Ähnlichkeitsmaßes der transformierten ersten Region des Atlasbilds und einer entsprechenden ersten Region des gegenständlichen Bilds;
- einen Auswertungsschritt (330) zum Auswerten der in Frage kommenden Transformation unter Verwendung eines auf dem berechneten Ähnlichkeitsmaß basierenden Kriteriums und Ermitteln einer optimalen Transformation basierend auf der Auswertung der in Frage kommenden Transformation;
- einen Erweiterungsschritt (340) zum Erweitern der optimalen Transformation der ersten Region des Atlasbilds auf eine zweite Region des Atlasbilds, wobei die zweite Region die erste Region umfasst, wodurch eine Registrierungstransformation geschaffen wird; und
- einen Registrierungsschritt (350) zum Transformieren der zweiten Region unter Verwendung der Registrierungstransformation, wodurch das Atlasbild mit dem gegenständlichen Bild registriert wird.

7. Computerprogrammprodukt, das durch eine Computeranordnung zu laden ist, umfassend Anweisungen zum Registrieren eines Atlasbilds aus einem Atlas multidimensionaler Bilder mit einem gegenständlichen Bild, wobei das Atlasbild basierend auf einer Vielzahl von Trainingsbildern berechnet wurde, wobei die Computeranordnung eine Verarbeitungseinheit und einen Speicher umfasst, wobei das Computerprogrammprodukt, nachdem es geladen wurde, die genannten Verarbeitungseinheit in die Lage versetzt, die Folgenden Aufgaben durchzuführen:
- Erzeugen einer in Frage kommenden Transformation basierend auf einem in Frage kommenden Transformationsfeld zum Transformieren einer ersten Region des Atlasbilds, wobei die erste Region eine Vielzahl von Orientierungspunkten umfasst, jedes Trainingsbild aus der Vielzahl von Trainingsbildern die Vielzahl von Orientierungspunkten umfasst, und wobei das in Frage kommende Transformationsfeld durch elementare Transformationsfelder definiert wird, die jeweils abgeleitet werden von Verlagerungen von i) der Vielzahl von Orientierungspunkten in einem jeweiligen der Vielzahl von Trainingsbildern relativ zu ii) der Orientierungspunktposition in dem Atlasbild, wobei die in Frage kommende Transformation eine lineare Kombination der elementaren Transformationsfelder ist, und wobei jedes elementare Transformationsfeld basierend auf der Hauptkomponentenanalyse der genannten Verlagerungen der Vielzahl von Orientierungspunkten berechnet wird;
- Transformieren der ersten Region des Atlasbilds unter Verwendung der in Frage kommenden Transformation;
- Berechnen eines Ähnlichkeitsmaßes der transformierten ersten Region des Atlasbilds und einer entsprechenden ersten Region des gegenständlichen Bilds;
- Auswerten der in Frage kommenden Transformation unter Verwendung eines auf dem berechneten Ähnlichkeitsmaß basierenden Kriteriums und Ermitteln einer optimalen Transformation basierend auf der Auswertung der in Frage kommenden Transformation;
- Erweitern der optimalen Transformation der ersten Region des Atlasbilds auf eine zweite Region des Atlasbilds, wobei die zweite Region die erste Region umfasst, wodurch eine Registrierungstransformation geschaffen wird; und
- Transformieren der zweiten Region unter Verwendung der Registrierungstransformation, wodurch das Atlasbild mit dem gegenständlichen Bild registriert wird.

## Revendications

1. Système (100) pour enregistrer une image d'atlas à partir d'un atlas d'images multidimensionnelles avec une image objective, l'image d'atlas ayant été calculée sur la base d'une pluralité d'images d'apprentissage, et le système comprenant :
- une unité de génération (105) pour générer une transformation candidate sur la base d'un domaine de transformation candidat pour transformer une première région de l'image d'atlas, la première région comprenant une pluralité de repères, chaque image d'apprentissage de la pluralité d'images d'apprentissage comprenant la pluralité de repères, et le domaine de transformation candidat étant défini par des domaines de transformation élémentaires, chacun étant dérivé de déplacements de i) la pluralité de repères dans une image respective de la pluralité d'images d'apprentissage, par rapport à ii) la position des repères sur l'image d'atlas, la transformation candidate étant une combinaison linéaire des domaines de transformation élémentaires, et chaque domaine de transformation élémentaire étant calculé sur la base de l'analyse des composants principaux desdits déplacements de la pluralité de repères ;
- une unité de transformation (110) pour transformer la première région de l'image d'atlas au moyen de la transformation candidate ;
- une unité de similitude (120) pour calculer une mesure de similitude de la première région transformée de l'image d'atlas et d'une première région correspondante de l'image objective ;
- une unité d'évaluation (130) pour évaluer la transformation candidate au moyen d'un critère basé sur la mesure de similitude calculée et déterminer une transformation optimale sur la base de l'évaluation de la transformation candidate ;
- une unité d'extension (140) pour étendre la transformation optimale de la première région de l'image d'atlas à une seconde région de l'image d'atlas, la seconde région comprenant la première région, créant ainsi une transformation d'enregistrement ; et
- une unité d'enregistrement (150) pour transformer la seconde région au moyen de la transformation d'enregistrement, enregistrant ainsi l'image d'atlas avec l'image objective.

2. Système (100) selon la revendication 1, dans lequel la mesure de similitude calculée est un maximum dans un espace de paramètres défini par les coefficients de la combinaison linéaire des domaines élémentaires.

3. Système (100) selon la revendication 1, comprenant en outre une unité d'atlas (155) pour construire l'atlas d'images multidimensionnelles.

4. Appareil d'acquisition d'images (400) comprenant un système (100) selon la revendication 1.

5. Poste de travail (500) comprenant un système (100) selon la revendication 1.

6. Procédé (300) d'enregistrement d'une image d'atlas à partir d'un atlas d'images multidimensionnelles avec une image objective, l'image d'atlas ayant été calculée sur la base d'une pluralité d'images d'apprentissage, et le procédé comprenant :
- une étape de génération (305) pour générer une transformation candidate sur la base d'un domaine de transformation candidat pour transformer une première région de l'image d'atlas, la première région comprenant une pluralité de repères, chaque image d'apprentissage de la pluralité d'images d'apprentissage comprenant la pluralité de repères, et le domaine de transformation candidat étant défini par des domaines de transformation élémentaires, chacun étant dérivé de déplacements de i) la pluralité de repères sur une image respective de la pluralité d'images d'apprentissage, par rapport à ii) la position des repères sur l'image d'atlas, la transformation candidate étant une combinaison linéaire des domaines de transformation élémentaires, et chaque domaine de transformation élémentaire étant calculé sur la base de l'analyse des composants principaux desdits déplacements de la pluralité de repères ;
- une étape de transformation (310) pour transformer la première région de l'image d'atlas au moyen de la transformation candidate ;
- une unité de similitude (320) pour calculer une mesure de similitude de la première région transformée de l'image d'atlas et d'une première région correspondante de l'image objective ;
- une étape d'évaluation (330) pour évaluer la transformation candidate au moyen d'un critère basé sur la mesure calculée de similitude et déterminer une transformation optimale sur la base de l'évaluation de la transformation candidate ;
- une étape d'extension (340) pour étendre la transformation optimale de la première région de l'image d'atlas à une seconde région de l'image d'atlas, la seconde région comprenant la première région, créant ainsi une transformation d'enregistrement ; et
- une étape d'enregistrement (350) pour transformer la seconde région au moyen de la transformation d'enregistrement, enregistrant ainsi l'image d'atlas avec l'image objective.

7. Produit de programme informatique devant être chargé par un dispositif informatique, comprenant les instructions pour enregistrer une image d'atlas à partir d'un atlas d'images multidimensionnelles avec une image objective, l'image d'atlas ayant été calculée sur la base d'une pluralité d'images d'apprentissage, le dispositif informatique comprenant une unité de traitement et une mémoire, le produit de programme informatique, une fois chargé, fournissant à ladite unité de traitement la capacité d'effectuer les tâches suivantes :
- la génération d'une transformation candidate sur la base d'un domaine de transformation candidat pour transformer une première région de l'image d'atlas, la première région comprenant une pluralité de repères, chaque image d'apprentissage de la pluralité d'images d'apprentissage comprenant la pluralité de repères, et le domaine de transformation candidat étant défini par des domaines de transformation élémentaires, dérivés chacun de déplacements de i) la pluralité de repères dans une image respective de la pluralité d'images d'apprentissage, par rapport à ii) la position des repères sur l'image d'atlas, la transformation candidate étant une combinaison linéaire des domaines de transformation élémentaires, et chaque domaine de transformation élémentaire étant calculé sur la base de l'analyse des composants principaux desdits déplacements de la pluralité de repères ;
- la transformation de la première région de l'image d'atlas au moyen de la transformation candidate ;
- le calcul d'une mesure de similitude de la première région transformée de l'image d'atlas et d'une première région correspondante de l'image objective ;
- l'évaluation de la transformation candidate au moyen d'un critère basé sur la mesure de similitude calculée et la détermination d'une transformation optimale sur la base de l'évaluation de la transformation candidate ;
- l'extension de la transformation optimale de la première région de l'image d'atlas à une seconde région de l'image d'atlas, la seconde région comprenant la première région, créant ainsi une transformation d'enregistrement ; et
- la transformation de la seconde région au moyen de la transformation d'enregistrement, enregistrant ainsi l'image d'atlas avec l'image objective.
